# EUROPÄISCHE PATENTSCHRIFT

(11) **EP 0 427 185 B1**
(45) Veröffentlichungstag und Bekanntmachung des Hinweises auf die Patenterteilung: **22.10.1997**
(21) Anmeldenummer: 90121163.1
(22) Anmeldetag: 06.11.1990
(51) Int. Cl.: C08G 63/64, A61L 17/00, A61K 47/34, A01N 25/10

(54) **Neue Copolymere aus Trimethylencarbonat und D,L-Lactid**
Copolymers from trimethylenecarbonate and D,L-lactide
Copolymères de carbonate de triméthylène et de D,L-lactide

(30) Priorität: 09.11.1989 DE 3937272
(43) Veröffentlichungstag der Anmeldung: 15.05.1991
(73) Patentinhaber: Boehringer Ingelheim KG, 55216 Ingelheim (DE); Boehringer Ingelheim International GmbH, 55216 Ingelheim (DE)
(72) Erfinder: Buchholz, Berthold, Dr., W-6507 Ingelheim am Rhein (DE)

(56) Entgegenhaltungen:
- EP-A- 0 372 221
- WO-A-88/10260
- US-A- 4 045 418

## Beschreibung

Die Erfindung betrifft neue Copolymere aus Trimethylencarbonat (1,3-Dioxan-2-on) und D,L-Lactid, ihre Herstellung und ihre Verwendung.

Die Verwendung von Trimethylencarbonat-Polyestern zur Herstellung einer Vielzahl von chirurgischen Gegenständen ist bekannt. In Verbindung damit ist bei dem Einsatz von Copolymeren des Trimethylencarbonats schon häufig versucht worden, durch die entsprechende Auswahl des bzw. der anderen Comonomeren die Eigenschaften der daraus resultierenden Polyester entsprechend dem geplanten Verwendungszweck zu modifizieren.

So sind aus dem Stand der Technik Copolymerisate von Trimethylencarbonat (TMC) mit Lactiden - wie Glycolid oder L-Lactid - bekannt.

Beispielsweise offenbart die US-PS 47 05 820 ein sogenanntes "Random-Polymerisat", das aus Trimethylencarbonat- und Glycolideinheiten aufgebaut ist.

Die US-PS 46 52 264 betrifft eine chirurgische Prothese, die aus einem bioabbaubaren Fasermaterial aufgebaut wird. Dabei bestehen die Fasern aus einem Copolymeren, das bis zu 50 % aus Trimethylencarbonat-Einheiten besteht, wobei als weitere Comonomere die o.g. Lactide eingesetzt werden.

In der US-PS 46 33 873 wird die Verwendung eines Polyesters aus Trimethylencarbonat- und Glycolideinheiten für die Herstellung eines elastischen chirurgischen Hilfsmittels beschrieben.

Die US-PS 44 29 080 hat bioabbaubare Fäden bzw. Gewebe zum Gegenstand, die aus einem Blockpolymeren hergestellt werden, das sich aus Trimethylencarbonat- und Glycolidblöcken zusammensetzt.

Die US-PS 43 00 656 bezieht sich auf chirurgische Gegenstände, die aus einem Copolymer hergestellt werden, das sich im wesentlichen aus Glycolideinheiten aufbaut. Als weiteres geeignetes Comonomer ist dort ebenfalls Trimethylencarbonat (TMC) beschrieben.

Desgleichen offenbart die Deutsche Offenlegungsschrift 28 50 824 ein Copolymer, welches aus Glycolid und Trimethylencarbonat (TMC) hergestellt wird, das ebenfalls bei der Herstellung von chirurgischen Befestigungselementen Verwendung findet.

Die Deutsche Patentschrift 28 21 570 offenbart ein Verfahren zur Herstellung derartiger Copolymerisate, in dem die einzelnen Monomeren bei der Polymerisation sequentiell zugefügt werden, um so im Ergebnis bestimmte Eigenschaften des Copolymerisats zu erreichen.

Daneben wird in der WO 88/10260 ein Verfahren zur Herstellung von meso-Lactid und dessen Verwendung zur Herstellung von Poly(meso-lactid-co-trimethylencarbonat) beschrieben.

Des weiteren beschreibt die EP-A-0 372 221 ein kontinuierliches Verfahren zur Herstellung von resorbierbaren Polyestern, worunter auch Poly(D,L-lactid-co-trimethylencarbonat) und Poly(meso-lactid-co-trimethylencarbonat) aufgeführt sind.

Aus den beiden letztgenannten Offenlegungsschriften sind jedoch keine weiteren Ausführungen zu Polymeren aus Trimethylencarbonat und den genannten optisch inaktiven lactiden zu entnehmen.

Überraschenderweise wurde nun gefunden, daß sich durch die Verwendung von D,L- lactid als Comonomeres bei der Copolymerisation mit Trimethylencarbonat (TMC), die Eigenschaften des dabei erhaltenen Copolymerisates auf einfachem Wege und in vielfältiger Weise beeinflussen lassen, ohne dabei die Anwendung spezieller Polymerisationstechniken oder Verfahrensmaßnahmen erforderlich zu machen.

Die neuen Copolymerisate auf der Basis von Trimethylencarbonat- (1,3-Dioxan-2-on-)Einheiten und D,L-Lactid-Einheiten weisen zwischen 30 und 90 Gew.-% Einheiten auf der Basis von D,L-Lactid auf. Das Copolymerisat kann ferner einen zusätzlichen kleineren Anteil von Einheiten eines weiteren Monomeren aus der Gruppe der Lactide oder Lactone aufweisen.

Wie ein Vergleich der Werte aus Tabelle 1 zeigt, können die mechanischen Eigenschaften der erfindungsgemäßen Copolymere durch die Variation der Anteile des eingesetzten Lactids und Trimethylencarbonats (TMC) in einem weiten Rahmen beeinflußt werden. So verkörpern Copolymerisate mit einem hohen Anteil (> 90 %) D,L-lactid Materialien mit vergleichsweise hoher Festigkeit. Im Vergleich zu reinem Poly(D,L-lactid zeichnen sich die erfindungsgemäßen Copolymerisate jedoch durch eine erhöhte Zähigkeit aus (kein Sprödbruch).

Polymere mit einem mittleren D,L-Lactid-Anteil (zwischen ca. 30 und 70 % D,L-Lactid) verkörpern außerordentlich dehnbare Materialien. Dabei durchlaufen die Werte für die Reißdehnung und für die Dehnung bei Maximalspannung mit sinkenden Lactidanteil ein Maximum (s. Tabelle 1).

Mit einem sinkenden Lactidanteil (< 30 %) ähneln die mechanischen Eigenschaften der Copolymerisate weitgehend reinem Polytrimethylencarbonat (Poly-TMC), daher werden Copolymerisate mit einem Lactid-Anteil > 30 % besonders bevorzugt. In Abhängigkeit von den angestrebten mechanischen Eigenschaften (fest und zäh oder elastisch) werden Polymere mit einem hohen oder mittleren Lactid-Anteil, die einem amorphen Charakter aufweisen, ganz besonders bevorzugt.

Die erfindungsgemäßen Copolymerisate können durch statistische Polymerisation (random) wie auch durch sequentielle Polymerisation hergestellt werden.

Die aus der Copolymerisation resultierenden bioabbaubaren Polymerisationsprodukte zeigen durchweg einen amorphen Charakter, wobei die Glasübergangstemperatur der erfindungsgemäßen Copolymerisate von dem Verhältnis von Trimethylencarbonat (TMC) zu. D,L-Lactid im Bereich von ca. -20°C bis ca. +55°C bevorzugt in einem Intervall von -15 bis + 50°C und besonders bevorzugt in einem Bereich von -13 bis +45°C - bestimmt mittels Differentialthermoanalyse mit einer Aufheizrate von 5 K/min. - liegt. Die Werte der inhärenten Viskosität der erfindungsgemäßen Copolymerisate liegen - gemessen in einer 0,1 %-igen Lösung in Chloroform bei 25°C - in einem Bereich von 0,5 bis 3 dl/g, bevorzugt in einem Intervall von 0,6 bis 2,5 dl/g und besonders bevorzugt in einem Bereich von 0,7 bis 2,1 dl/g. In Abhängigkeit von der jeweiligen Glasübergangstemperatur weisen die erfindungsgemäßen Copolymerisate bestimmte Elastizitätsmerkmale auf, die sie zum Einsatz für eine Vielzahl von verschiedenen Anwendungsformen geeignet erscheinen lassen.

Ferner lassen sich durch geeignete Wahl der Reaktionsbedingungen Molmasse und die Zusammensetzung des Copolymerisates sowie die Sequenz der Trimethylencarbonat- bzw. Lactideinheiten beeinflussen. Durch die Beeinflussung der Sequenz der Trimethylencarbonat- und Lactideinheiten läßt sich ferner eine Modifizierung der in vivo Eigenschaften der aus den Copolymerisaten hergestellten chirurgischen Gegenständen erzielen.

Weitere Möglichkeiten, bestimmte Charakteristika der resultierenden Copolymerisate zu modifizieren, bestehen z.B. in der Zugabe von Weichmachern oder anderen geeigneten - aus dem Stand der Technik bekannten - polymeren Additiven.

Aufgrund ihrer elastischen Eigenschaften eignen sich die erfindungsgemäßen Copolymerisate besonders zum Einsatz in Form von:
1) Beschichtungsmaterial für chirurgisches Nahtmaterial oder Befestigungselemente. Derartige Beschichtungen erhöhen z.B. die Gleitfähigkeit von Nahtmaterial und verleihen diesem bessere Verknotungseigenschaften.
   Die erfindungsgemäßen Copolymerisate können auf einfachem Wege auf die betreffenden chirurgischen Hilfsmittel aufgebracht werden - z.B. durch Auftragen in Form einer Lösung oder durch Auftragen unter Anwendung thermischer Verfahren. Beide Methoden sind aus dem Stand der Technik bekannt.
   Dabei wirkt sich besonders vorteilhaft aus, daß die Copolymerisate in zahlreichen Lösungsmitteln löslich sind. Als Lösungsmittel eignen sich beispielsweise neben chlorierten Kohlenwasserstoffen auch physiologisch unbedenkliche Lösungsmittel wie z.B. Aceton, Essigsäureethylester und Propylencarbonat (4-Methyl-1,3-dioxolan-2-on).
2) resorbierbaren Folien zur Wundabdeckung z.B. zur Behandlung von Brandwunden oder Oberflächenabschürfungen. Zu diesem Zweck können die erfindungsgemäßen Copolymerisate gegebenenfalls auch als Trägermaterialien für Pharmazeutika - wie z.B. entzündungshemmende oder bakterizide Mittel - dienen; es ist jedoch auch der Einsatz anderer Arzneimittel denkbar.
   Bei derartigen Verletzungen der Haut war es bislang erforderlich, einen Verband anzulegen, wobei stets Vorkehrungen getroffen werden mußten, um das Einwachsen des nicht absorbierbaren Verbandes in das sich neu bildende Gewebe zu verhindern.
   Da die erfindungsgemäßen Copolymerisate resorbierbar sind, wenn Teile der Bandage in die Regenerationsebene hineinreichen, regeneriert sich das Gewebe und absorbiert dabei das Copolymerisat, während das nicht resorbierte Copolymer mit dem Schorf abgestoßen werden kann.
   Aufgrund ihrer Elastizität und ihrem Lösungsverhalten sind die erfindungsgemäßen Verbindungen im besonderen Maße auch als Sprühpflaster - zur Wundabdeckung kleinerer Wunden - geeignet.
3) elastischen resorbierbaren Implantaten:
   So können die erfindungsgemäßen Copolymerisate beispielsweise als Kissen, Bandagen oder Schwämme eingesetzt werden, die bei chirurgischen Eingriffen Verwendung finden. Derartige chirurgische Hilfsmittel werden bei einer Vielzahl von chirurgischen Eingriffen benötigt. Die Verwendung dieser Hilfsmittel erweist sich dann als besonders vorteilhaft, wenn die Absicht besteht, diese Hilfsmittel während oder nach dem chirurgischen Eingriff zu entfernen, bei denen jedoch durch ein Versehen oder durch einen Zufall ein Teil zurückbleiben kann. So besteht eine der Komplikationen, die bei Operationen auftreten können, darin, daß Baumwollfasern von Baumwollschwämmen oder -tupfern, in der Wunde zurückbleiben können. Bei der Verwendung der entsprechenden Hilfsmittel aus den erfindungsgemäßen Copolymerisaten werden dagegen alle Fragmente, die sich ablösen, ohne nachteilige Folgen resorbiert.
   Durch den Sachverhalt, daß über die Zusammensetzung bzw. über die Sequenz der einzelnen Comonomere Einfluß auf die "in vivo"-Eigenschaften - d.h. auch auf das Abbauverhalten - der Copolymere ohne große Mühe Einfluß genommen werden kann, sind diese insbesondere auch für solche Anwendungsformen interessant, bei denen der vorübergehende Einsatz einer Prothese oder Bandage erforderlich ist, wie z.B. Vorrichtungen zur Hämostasis Gesichtsplastiken, Flecke für Eingeweidebrüche (Hernien), Dentalpackungen etc.
4) Trägern oder Behältnissen für Pharmazeutika bzw. als Retardierungshilfsstoffe für Wirkstoffreigabesysteme, bei denen eine kontrollierte Freigabe des Arzneimittels angestrebt wird.
   Bei derartigen Wirkstoffreigabesystemen ist es erwünscht, daß sich das Behälter- bzw. Trägermaterial während oder nach der Freigabe des Wirkstoffs auflöst und dabei keine unerwünschten oder gar pharmakologisch bedenklichen Rückstände im Gewebe zurückläßt. So gestaltete Freigabesysteme können nicht nur als Implantat sondern auch für eine orale Applikation oder für eine Verabreichung in Form eines Aerosols konzipert werden.
   Möglichkeiten, die erfindungsgemäßen Copolymerisate mit Wirkstoff zu beladen, bzw. das Wirkstoffreigabesystem von vornherein so zu gestalten, daß eine gewünschte Freigabecharakteristik erzielt wird, sind aus dem Stand der Technik bekannt.
   Weiterhin ist es möglich, die erfindungsgemäßen Copolymerisate als Träger oder Behältnisse u.a. für Agrochemikalien, Herbizide oder Insektizide einzusetzen.
   Aufgrund ihrer Elastizitätseigenschaften eignen sich die erfindungsgemäßen Copolymerisate z.B. für eine Anwendung in Form von Folien oder Pflastern zur Verwendung in transdermalen Wirkstoffreigabesystemen.
   Möglichkeiten, die erfindungsgemäßen Copolymerisate als Wirkstoffreigabesysteme, die z.B. in Form von Implantanten oder Boli eingesetzt werden, zu konzipieren, sind ebenfalls aus dem Stand der Technik bekannt.

Die Verfahren zur Herstellung der erfindungsgemäßen Copolymerisate als "Random-" oder Blockpolymerisate und der chirurgischen Gegenstände erfolgen in üblicher Weise - hierzu wird u.a. auf den obengenannten Stand der Technik verwiesen. In ähnlicher Weise werden auch die hiernach erhaltenen chirurgischen Gegenstände eingesetzt. Die nachfolgenden Beispiele sollen die Erfindung erläutern, ohne sie einzuschränken:

### Beispiel 1

2.5 g Trimetyhlencarbonat, 2.5 g D,L-Lactid und 0.25 ml einer Lösung von Zinnoctoat (Zinn(II)-2-ethylhexanoat) in Toluol (136.5 mg in 20 ml) werden nacheinander in ein Glasröhrchen gefüllt. Zum Entfernen des Lösungsmittels wird mehrmals evakuiert. Das Glasröhrchen wird unter Stickstoffatmosphäre zugeschmolzen, in ein auf 190°C temperiertes Ölbad eingehängt und dort 2.5 Stunden belassen. Nach dem Abkühlen auf Raumtemperatur wird das Glasröhrchen zerbrochen und anhaftende Glasreste vom Polymeren entfernt. Zum Entfernen von nicht umgesetzten Monomeren wird das Polymer aus Chloroform/Petrolether umgefällt und i.Vak. getrocknet.

Die NMR-spektroskopische Untersuchung (250 MHz-¹H-NMR, CDCl₃) ergibt einen Umsetzungsgrad von 96 % für D,L-Lactid und von 97 % für Trimethylencarbonat. Das Copolymer besteht zu 51 Gewichtsprozent aus Trimethylencarbonateinheiten. Das Rohprodukt besitzt eine inhärente Viskosität von 1.05 dl/g (gemessen in einer 0.1 %-igen Lösung in Chloroform bei 25°C). Das gereinigte Polymer weist eine Glasübergangstemperatur von +9°C auf (DSC, Aufheizrate 5 K/min.).

### Beispiele 2 bis 13

Entsprechend der Versuchsdurchführung in Beispiel 1 werden statistische Copolymere aus Trimethylencarbonat und D,L-Lactid in verschiedener Zusammensetzung hergestellt:

| Beispiel | Ansatzgröße | eingesetze Menge Trimethylencarbonat | inhärente Viskosität ¹⁾ |
|---|---|---|---|
| 2 | 5 g | 5% | 1.23 |
| 3 | 5 g | 10 % | 1.36 |
| 4 | 100 g | 13 % | 1.68 |
| 5 | 5 g | 20 % | 1.11 |
| 6 | 100 g | 31 % | 1.17 |
| 7 | 5 g | 40 % | 0.86 |
| 8 | 100 g | 50 % | 1.15 |
| 9 | 5 g | 60 % | 1.15 |
| 10 | 100 g | 69 % | 1.13 |
| 11 | 5 g | 80 % | 1.23 |
| 12 | 100 g | 88 % | 1.27 |
| 13 | 5 g | 95 % | 1.82 |

| | | | |
|---|---|---|---|
| ¹⁾ Angaben in dl/g gemessen in einer 0.1 %-igen Lösung in Chloroform bei 25°C. Die Werte beziehen sich bei den 5 g-Ansätzen auf die Rohprodukte, bei den 100 g-Ansätzen auf die gereinigten Polymere. | | | |

Analytische Daten zu den nach den Beispielen 2 bis 13 hergestellten Copolymerisaten:

| Beispiel | Umsetzungsgrad | | Polymerzusammens. Gew.% TMC-Einheit. | Glasübergangstemperatur [°C] |
|---|---|---|---|---|
| | TMC¹⁾ | Lactid | | |
| 2 | 25 % | 94 % | 4 % | |
| 3 | 55 % | 94 % | 6 % | + 41 |
| 4 | 65 % | 95 % | 9 % | |
| 5 | 84 % | 95 % | 17 % | + 26 |
| 6 | 92 % | 96 % | 30 % | + 23 |
| 7 | 95 % | 96 % | 42 % | |
| 8 | 97 % | 97 % | 50 % | + 9 |
| 9 | 98 % | 96 % | 62 % | |
| 10 | 98 % | 98 % | 70 % | - 2 |
| 11 | 98 % | 2) | | |
| 12 | 98 % | 2) | 90 % | - 13 |
| 13 | 98 % | 2) | | |

| | | | | |
|---|---|---|---|---|
| ¹⁾ TMC : Trimethylencarbonat | | | | |
| ²⁾ Bei den Beispielen 11 bis 13 läßt sich der Lactid-Umsatz ¹H-NMR-spektroskopisch nicht mit einer ausreichenden Genauigkeit bestimmen. | | | | |

### Beispiel 14

Blockpolymerisat aus Trimethylencarbonat und D,L-Lactid:

95 g Trimethylencarbonat und 1.90 ml einer Lösung von 853 mg Zinnoctoat in 50 ml Toluol werden nacheinander in einen Glaskolben gefüllt. Zum Entfernen des Lösungsmittels wird mehrmals evakuiert und anschließend mit Stickstoff belüftet. Der Kolben wird in ein auf 190°C temperiertes ölbad eingehängt. Während der Polymerisation wird mit einem Edelstahlrührer gerührt und mit einem leichten Stickstoffstrom begast. Nach zwei Stunden werden 190 g D,L-Lactid zugefügt. Nach weiteren zwei Stunden wird der hochviskose Kolbeninhalt ausgegossen und erkalten gelassen.

Die NMR-spektroskopische Untersuchung (250 MHz-¹H-NMR, CDCl₃) ergibt einen Umsetzungsgrad von 44 % für D,L-Lactid und von 97 % für Trimethylencarbonat.

Zum Entfernen von nicht umgesetzten Monomerenanteilen wird das Rohprodukt zweimal aus Chloroform/Petrolether umgefällt. Das gereinigte Polymer besitzt eine inhärente Viskosität von 0.77 dl/g (gemessen in einer 0.1 %-igen Lösung in Chloroform bei 25°C) und besteht zu 54 Gew.-% Mol-% aus Trimethylencarbonateinheiten (250 MHz-¹H-NMR).

**Tabelle 1**

| Zugversuche an Poly(D,L-lactid-co-TMC) (durchgeführt bei Raumtemperatur an schmelzgepressten Probekörpern der Dimension 180 x 15 x 2 mm) | | | | | |
|---|---|---|---|---|---|
| Material | Zusammensetzung | ^{σ}zM¹⁾ | ^{σ}zR²⁾ | ^{ε}zM³⁾ | ^{ε}zR⁴⁾ |
| P-TMC⁵⁾ | | 1,3 | 0,9 | 85 | 193 |
| P-(TMC/DL-LA)⁶⁾ | 90/10 | 1,2 | 1,0 | 84 | 166 |
| P-(TMC/DL-LA) | 70/30 | 1,6 | 1,2 | 104 | 226 |
| P-(TMC/DL-LA) | 50/50 | 5,2 | 5,2 | 963 | 963 |
| P-(TMC/DL-LA) | 30/70 | 14 | 14 | 519 | 519 |
| P-(TMC/DL-LA) | 10/90 | 27 | 11 | 4,3 | 28 |
| P-DL-LA⁷⁾ | | 52 | 52 | 3,6 | 3,6 |

| | | | | | |
|---|---|---|---|---|---|
| 1) ^{σ}zM = Zugfestigkeit [N/mm²] | | | | | |
| 2) ^{σ}zR = Reißfestigkeit [N/mm²] | | | | | |
| 3) ^{ε}zM = Dehnung bei Maximalspannung [%] | | | | | |
| 4) ^{ε}zR = Reißdehnung [%] | | | | | |
| 5) P-TMC = Polytrimethylencarbonat | | | | | |
| 6) P-(TMC/DL-LA)= Poly(trimethylencarbonat-co-D,L-lactid) | | | | | |
| 7) P-DL-LA = Poly-D,L-lactid | | | | | |

## Patentansprüche

1. Copolymer auf der Basis von Trimethylencarbonat- und D, L-Lactid-Einheiten, dadurch gekennzeichnet, daß es einen Gehalt an D,L-Lactid-Einheiten im Bereich von 30 bis 90 Gew.-% aufweist.

2. Copolymer nach Anspruch 1, dadurch gekennzeichnet, daß es einen zusätzlichen Anteil von Einheiten eines weiteren Monomeren aus der Gruppe der Lactide oder Lactone aufweist.

3. Copolymer nach einem der Ansprüche 1 bis 2, dadurch gekennzeichnet, daß es Werte der inhärenten Viskosität - gemessen in einer 0,1 %igen Lösung in Chloroform bei 25°C - aufweist, die in einem Bereich von 0,5 bis 3 dl/g liegen.

4. Copolymer nach Anspruch 3, dadurch gekennzeichnet, daß es Werte der inhärenten Viskosität - gemessen in einer 0,1 %igen Lösung in Chloroform bei 25°C - aufweist, die in einem Bereich von 0,6 bis 2,5 dl/g liegen.

5. Copolymer nach Anspruch 4, dadurch gekennzeichnet, daß es Werte der inhärenten Viskosität - gemessen in einer 0,1 %igen Lösung in Chloroform bei 25°C - aufweist, die in einem Bereich von 0,7 bis 2,1 dl/g liegen.

6. Copolymer nach einem der Ansprüche 1 oder 2, dadurch gekennzeichnet, daß es eine Glasübergangstemperatur - bestimmt mittels Differentialthermoanalyse mit einer Aufheizrate von 5 K/min. - aufweist, die in einem Bereich von -20 bis +55°C liegt.

7. Copolymer nach Anspruch 6, dadurch gekennzeichnet, daß es eine Glasübergangstemperatur - bestimmt mittels Differentialthermoanalyse mit einer Aufheizrate von 5 K/min. - aufweist, die in einem Bereich von -15 bis + 50°C liegt.

8. Copolymer nach Anspruch 7, dadurch gekennzeichnet, daß es eine Glasübergangstemperatur - bestimmt mittels Differentialthermoanalyse mit einer Aufheizrate von 5 K/min. - aufweist, die in einem Bereich von -13 bis +45°C liegt.

9. Verfahren zur Herstellung eines Copolymeren gemäß einem der Ansprüche 1 bis 8, dadurch gekennzeichnet, daß man Trimethylencarbonat und D,L-Lactid in Gegenwart eines Katalysators polymerisiert und das Produkt isoliert.

10. Verfahren zur Herstellung eines Copolymeren nach Anspruch 9, dadurch gekennzeichnet, daß kleinere Mengen eines weiteren Monomeren aus der Gruppe der Lactide oder Lactone zugefügt werden.

11. Verwendung eines Copolymeren gemäß einem der Ansprüche 1 bis 8 als Beschichtungsmaterial für chirurgische Nahtmaterial oder für chirurgische Befestigungselemente.

12. Verwendung eines Copolymeren gemäß einem der Ansprüche 1 bis 8 als resorbierbare Folie zur Wundabdeckung oder in Form eines Sprühpflaster-Verbandes.

13. Verwendung eines Copolymeren gemäß einem der Ansprüche 1 bis 8 als resorbierbares chirurgische Implantat.

14. Verwendung eines Copolymeren gemäß einem der Ansprüche 1 bis 8 als Träger, Matrix oder Behältnis für pharmazeutische Wirkstoffe.

15. Verwendung eines Copolymeren gemäß einem der Ansprüche 1 bis 8 als Träger oder Behältnis für Agrochemikalien, Insektizide oder Herbizide.

## Claims

1. Copolymer based on trimethylenecarbonate and D,L-lactide units, characterised in that it contains an amount of D,L-lactide units ranging from 30 to 90% by weight.

2. Copolymer according to claim 1, characterised in that it contains an additional amount of units of another monomer selected from the group of lactides or lactones.

3. Copolymer according to one of claims 1 and 2, characterised in that it has values for inherent viscosity - measured in a 0,1% solution in chloroform at 25°C - in the range from 0.5 to 3 dl/g.

4. Copolymer according to claim 3, characterised in that it has values for inherent viscosity - measured in a 0.1% solution in chloroform at 25°C - in the range from 0.6 to 2.5 dl/g.

5. Copolymer according to claim 4, characterised in that it has values for inherent viscosity - measured in a 0.1% solution in chloroform at 25°C - in the range from 0.7 to 2.1 dl/g.

6. Copolymer according to one of claims 1 and 2, characterised in that it has a glass transition temperature - determined by differential thermoanalysis with a heating rate of 5 K/min. - in the range from -20 to +55°C.

7. Copolymer according to claim 6, characterised in that it has a glass transition temperature - determined by differential thermoanalysis with a heating rate of 5 K/min. - in the range from -15 to +50°C.

8. Copolymer according to claim 7, characterised in that it has a glass transition temperature - determined by differential thermoanalysis with a heating rate of 5 K/min. - in the range from -13 to +45°C.

9. Process for preparing a copolymer according to one of claims 1 to 8, characterised in that trimethylenecarbonate and D,L-lactide are polymerised in the presence of a catalyst and the product is isolated.

10. Process for preparing a copolymer according to claim 9, characterised in that minor amounts of another monomer selected from the group comprising the lactides or lactones are added.

11. Use of a copolymer according to one of claims 1 to 8 as a coating material for surgical suturing material or for surgical fixing elements.

12. Use of a copolymer according to one of claims 1 to 8 as a resorbable film for wound covering or in the form of a spray dressing.

13. Use of a copolymer according to one of claims 1 to 8 as a resorbable surgical implant.

14. Use of a copolymer according to one of claims 1 to 8 as a carrier, matrix or container for pharmaceutical active substances.

15. Use of a copolymer according to one of claims 1 to 8 as a carrier or container for agrochemicals, insecticides or herbicides.

## Revendications

1. Copolymère à base d'unités de carbonate de triméthylène et d'unités de D,L-lactide caractérisé en ce qu'il présente une teneur en unités de D,L-lactide dans le domaine de 30 à 90% en masse.

2. Copolymère selon la revendication 1 caractérisé en ce qu'il présente une proportion supplémentaire d'unités d'un autre monomère du groupe des lactides ou des lactones.

3. Copolymère selon l'une des revendications 1 à 2 caractérisé en ce qu'il présente des valeurs de viscosité inhérente, mesurées dans une solution à 0,1% dans le chloroforme à 25°C, qui sont situées dans un domaine de 0,5 à 3 dl/g.

4. Copolymère selon la revendication 3 caractérisé en ce qu'il présente des valeurs de viscosité inhérente, mesurées dans une solution à 0,1% dans le chloroforme à 25°C, qui sont situées dans un domaine de 0,6 à 2,5 dl/g.

5. Copolymère selon la revendication 4 caractérisé en ce qu'il présente des valeurs de viscosité inhérente, mesurées dans une solution à 0,1% dans le chloroforme à 25°C, qui sont situées dans un domaine de 0,7 à 2,1 dl/g.

6. Copolymère selon l'une des revendications 1 ou 2 caractérisé en ce qu'il présente une température de transition vitreuse, déterminée par analyse thermique différentielle avec une vitesse de chauffe de 5 K/min, qui est située dans un domaine de -20 à +55°C.

7. Copolymère selon la revendication 6 caractérisé en ce qu'il présente une température de transition vitreuse, déterminée par analyse thermique différentielle avec une vitesse de chauffe de 5 K/min, qui est située dans un domaine de -15 à +50°C.

8. Copolymère selon la revendication 7 caractérisé en ce qu'il présente une température de transition vitreuse, déterminée par analyse thermique différentielle avec une vitesse de chauffe de 5 K/min, qui est située dans un domaine de -13 à +45°C.

9. Procédé de préparation d'un copolymère selon l'une des revendications 1 à 8 caractérisé en ce que l'on polymérise du carbonate de triméthylène et du D,L-lactide en présence d'un catalyseur et l'on isole le produit.

10. Procédé de préparation d'un copolymère selon la revendication 9 caractérisé en ce que de plus petites quantités d'un autre monomère du groupe des lactides ou des lactones sont ajoutées.

11. Utilisation d'un copolymère selon l'une des revendications 1 à 8 comme matériau de revêtement pour matériel de suture chirurgical ou pour éléments de fixation chirurgicaux.

12. Utilisation d'un copolymère selon l'une des revendications 1 à 8 comme feuille résorbable pour le recouvrement des plaies ou sous forme d'un pansement d'emplâtre à atomisation.

13. Utilisation d'un copolymère selon l'une des revendications 1 à 8 comme implant chirurgical résorbable.

14. Utilisation d'un copolymère selon l'une des revendications 1 à 8 comme support, matrice ou récipient pour principes actifs pharmaceutiques.

15. Utilisation d'un copolymère selon l'une des revendications 1 à 8 comme support ou récipient pour produits agrochimiques, insecticides ou herbicides.
